# EUROPEAN PATENT APPLICATION

(11) **EP 4 190 380 A1**
(43) Date of publication of application: **07.06.2023**
(21) Application number: 21848616.5
(22) Date of filing: 12.07.2021
(51) Int. Cl.: A61M 16/00

(54) **VENTILATOR**

(30) Priority: 29.07.2020 ES 202031685 U
(71) Applicant: Fundació Eurecat, 08290 Cerdanyola del Vallès (Barcelona) (ES)
(72) Inventor: MAGALLON VALLES, Jose, 08760 MARTORELL (Barcelona) (ES); SÁNCHEZ VALENZUELA, Rafael, 08640 OLESA DE MONTSERRAT (Barcelona) (ES)
(74) Representative: Herrero & Asociados, S.L.
(86) International application number: PCT/ES2021/070505
(87) International publication number: WO 2022/023595

(57) **Abstract**

The respirator comprises an air bag that is pressurized to provide air to a user, wherein the air bag is a tubular membrane (2) housed within a pressure chamber (1). Furthermore, the pressure chamber (1) comprises fluid inlets and outlets, the fluid pressurizing the tubular membrane (2) to provide air to a user, and protrusions (9) that pressurize the tubular membrane (2).

It provides a respirator with reduced dimensions, number of parts, weight, and cost, as well as reusable after autoclave disinfection of the auto-inflatable bag as a standard element.

## Description

The present invention relates to a respirator of the type normally used for artificial respiration in persons who are unable to breathe on their own.

### Background of the invention

In the new normal healthcare system, the traditional mouth-to-mouth resuscitation of cardiorespiratory arrests is to be avoided at all costs, as it is forbidden due to the risk of infectious diseases due to COVID-19.

The use of respirators is widespread in hospitals, in ICU departments where there are critically ill patients, and also emergency ambulances carry respirators for patients with respiratory problems.

These respirators are mostly composed of housings that contain a hand-held resuscitator or self-inflating bag type AMBU (Airway Mask Bag Unit).

These devices provide positive pressure ventilation for patients who are not breathing or not breathing adequately. It is an essential part of any emergency equipment and is also frequently used in hospitals as an indispensable part of crash cart and emergency room equipment.

Respirators are also used within hospitals for temporary ventilation of patients dependent on a mechanical ventilator when this needs to be examined for possible malfunction, or when ventilator-dependent patients are transported within the hospital.

These respirators can be manual if used for a short period of time, during which a person presses the bag at set intervals to provide air to the user, or automated, if used for long periods of time.

Automated resuscitator devices comprise an actuation device that moves at predetermined intervals in an open position and a closed position. In the closed position, the mechanism presses the bag to provide air to the user, and in the open position it allows air into the bag to be provided in the next cycle to the user.

Some of these respirators have the disadvantage that the electric driving motors produce parasitic electrical interference in the sensitive electronics of intensive care units.

### Description of the invention

Therefore, an objective of the present invention is to provide a respirator of reduced dimensions, number of parts, weight, and cost, as well as reusable after autoclave disinfection of the auto-inflatable bag as a standard element. The aim of the invention is to provide a portable, hands-free, self-contained respirator apparatus for emergency medical care and that the source of energy is compressed air.

The respirator according to the present invention is defined by the independent claim. Additional features are defined in the dependent claims.

In the respirator according to the present invention, the power source may be portable, such as the oxygen cylinder carried by the medical assistant in the ambulance, in the emergency backpack, or at a fixed point such as the medical air intake at the head of the hospital bed.

Furthermore, with the respirator according to the present invention, because of the way in which we force the air bag membrane to collapse, the volume of air that can be delivered to the patient is approximately 30 % higher than the volume delivered by two-handed manual pumping.

It should be noted that in order to optimize the ventilation, the patient's lower jaw should be kept open and thus the airway should be maintained.

To this end, in its least invasive form, a palatal model diving regulator mouthpiece is used, which should be clamped between the patient's teeth.

This mouthpiece has a tube with a specific curvature and variable cross-sections attached to it by means of an adaptor, which allows the air to be taken directly into the oropharynx and, at the same time, holds the tongue against the palate, preventing it from falling due to relaxation, thus blocking the airway.

This can be done because the patient's unconsciousness overrides the gag reflex.

The patient, on regaining semiconsciousness and the gag reflex, will have convulsions on noticing a foreign body, and these signs will serve as a warning that semiconsciousness has returned.

With a cannula that is less invasive than the Guedel cannula and easier to insert into the patient's throat, it provides the option for use by less qualified healthcare personnel, allowing a wider range of personnel to use it.

This respirator, because of the way it is to be used in emergencies, uses a mask with an inflatable cushion and a harness to attach it to the patient's head.

This allows for an optimal facial fit, and it also has an anti-choking valve so that it performs the exhalation maneuver on its own. This allows it to function as a hands-free AMBU.

It should be noted that in this mask, air can only escape through the exhalation valve when we stop injecting air from the AMBU and perform cardiac massage.

By wearing the mask with a fastening harness, we stop putting pressure on the lower jaw, which can cause the head to return to its flat position, closing the pharynx.

In the respirator according to the present invention there are no gears to lubricate, no bearings, no track rollers, no shafts, no cams, no spindles, no levers, no moving parts to be protected, no electric motors that heat up and that heat up the surrounding environment, and no interference with adjacent electronic equipment.

### Description of the drawings

In order to complete the description being made and in order to assist in a better understanding of the features of the present invention, according to a preferred example of a practical embodiment of the same, a set of drawings is attached as an integral part of the said description, in which the following is illustrated for illustrative purposes and without limitation.
Figure 1A is an elevation view in longitudinal section of the respirator according to the present invention, according to a first embodiment;
Figure 1B is an elevation view in longitudinal section of the respirator according to the present invention, according to a second embodiment;

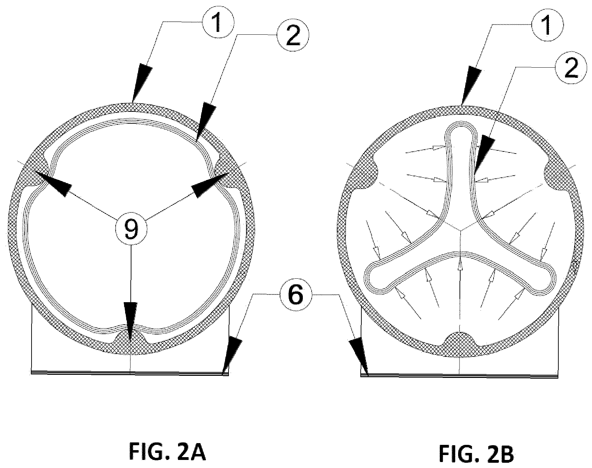

Figure 2A is a cross-sectional view of the respirator according to the present invention with the membrane in the rest position;
Figure 2B is a cross-sectional view of the respirator according to the present invention with the membrane in the fully controlled collapse position;

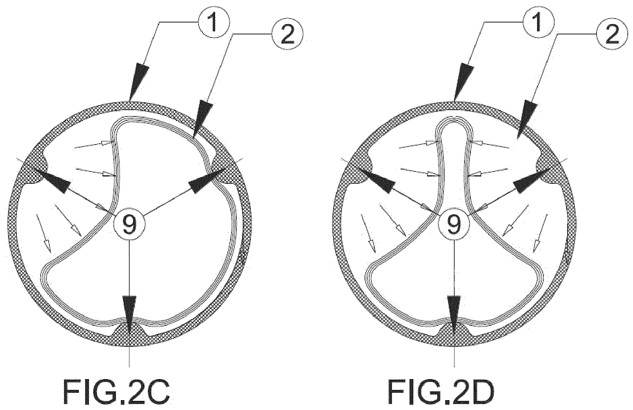

Figure 2C is a cross-sectional view of the respirator according to the present invention with the membrane in a controlled collapse position at one third of its capacity;
Figure 2D is a cross-sectional view of the respirator according to the present invention with the membrane in controlled collapse position at two-thirds of its capacity;
Figure 3 is a plan view in section of the control panel of the respirator according to the present invention with its component parts;
Figure 4 is a front view of the control panel of the respirator according to the present invention with its indicators;
Figure 5 is a rear view of the control panel of the respirator according to the present invention with the electrical and air intakes;
Figure 6A is an elevation view of a cannula used with the respirator according to the present invention;
Figure 6B is a plan view of the cannula of Figure 6A;
Figure 6C is a sectional view of sections A-A, B-B and C-C shown in Figure 6; and
Figure 7 is a sectional view showing the detail of the internal protrusions of the sealed pressure chamber.

### Description of a preferred embodiment

As shown in the embodiment depicted, the respirator according to the present invention comprises a sealed pressure chamber 1 and a control panel 10.

Inside the sealed pressure chamber 1 is a tubular membrane 2, type AMBU, with two non-return valves 4, an upstream non-return valve and a downstream non-return valve, positioned in such a way that the air can only flow in one direction.

The upstream non-return valve 4 includes a relief or overpressure valve 8, which prevents the patient from receiving an overpressure that could damage the lungs.

The inner part of the pressure chamber 1 is provided with fluid inlets and outlets 7, e.g., it also has protrusions 9, which, for example, are different from each other. The low-pressure compressed medical air introduced into the chamber 1 exerts pressure on the tubular membrane 2 and forces the membrane 2 to deform in a predetermined shape. In the embodiment shown, this predetermined shape is a tricuspid shape, although any suitable shape could be provided.

Furthermore, these protrusions 9 physically press the tubular membrane 2 by dimensional interference, giving it a predetermined shape. In other words, the pressure on the tubular membrane can be exerted by the fluid coming from the outlets and/or by the protrusions 9.

That is, the pressure on the tubular membrane 2 is exerted:
- first by physical interference between the membrane 2 and the internal protrusions 9 with the aim of breaking the tubular structure;
- second by fluid from the inlets and outlets 7, this fluid being responsible for the collapse of the lobes of the membrane 2.

At the air inlet end of the membrane 2 and on its outer side outside the natural air passage circuit to the patient is a flat transverse membrane 5 made of elastic material which, by means of a flange 6 or a quick-release flange 3.1 and screws, is pressed against the sealed chamber 1, fixing the membrane 2 in a sealed way to the pressure chamber 1.

This seals the pressure chamber 1 on this side and, at the same time, absorbs the differences in elongation of the tubular membrane 2 that occur when it collapses and expands. It also allows any overpressure inside the pressure chamber 1 to be absorbed, acting as a diaphragm.

An extension of this flange 6 acts as a support or legs for the respirator body.

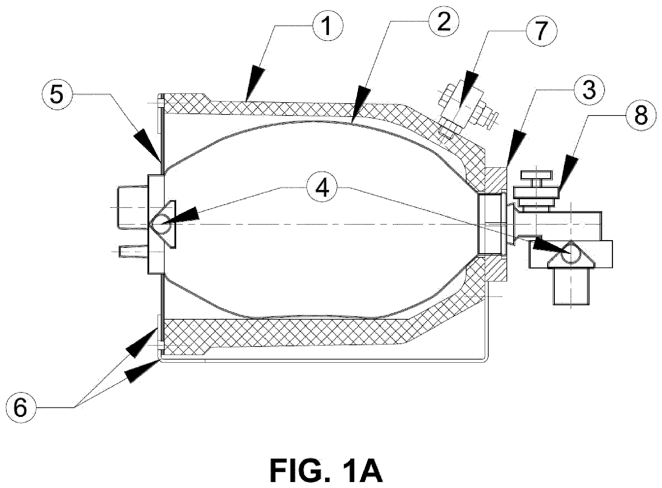

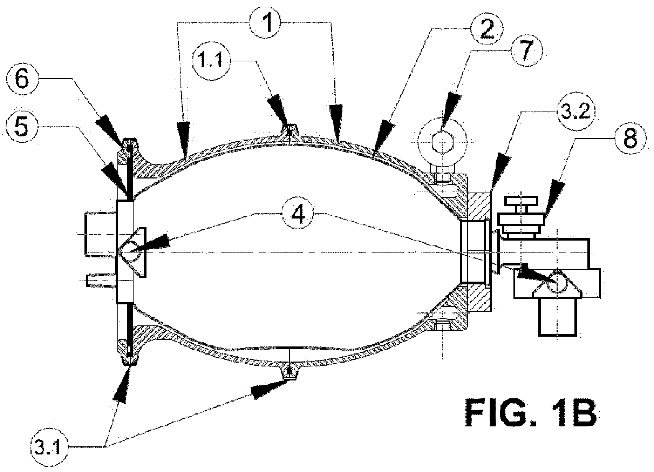

In the embodiment shown in figure 1B, a quick-release flange 3.1 presses two half-bodies of the sealed chamber 1 in its central part and seals the chamber 1 through an O-ring 1.1.

At the front of the pressure chamber 1 there are two half-flanges 3 (Figure 1A), or a quick-opening flange 3.2, which fix the tubular membrane 2 in this part and seal the pressure chamber 1 at this end.

The tubular membrane 2 is subjected to a controlled pressure for a controlled time and at a controlled flow rate, by means of a control panel 10 and a number of cycles per minute.

The way in which the tubular membrane 2 collapses is related to the period of time during which we subject it to pressure, to the pressure to which it is subjected, and to the air flow rate that we inject between the tubular membrane 2, the pressure chamber 1 and to the shape of the protrusions 9 located in the pressure chamber 1. From this control of the flow rate, pressure and time, the collapse of the membrane 2 by one third, two thirds or in its entirety is derived.

The air supply to the patient is directly related to the deformation of the tubular membrane 2 controlled through the control panel 10.

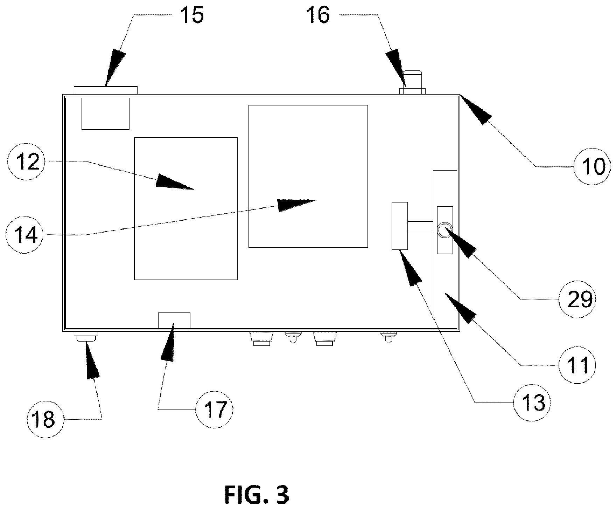

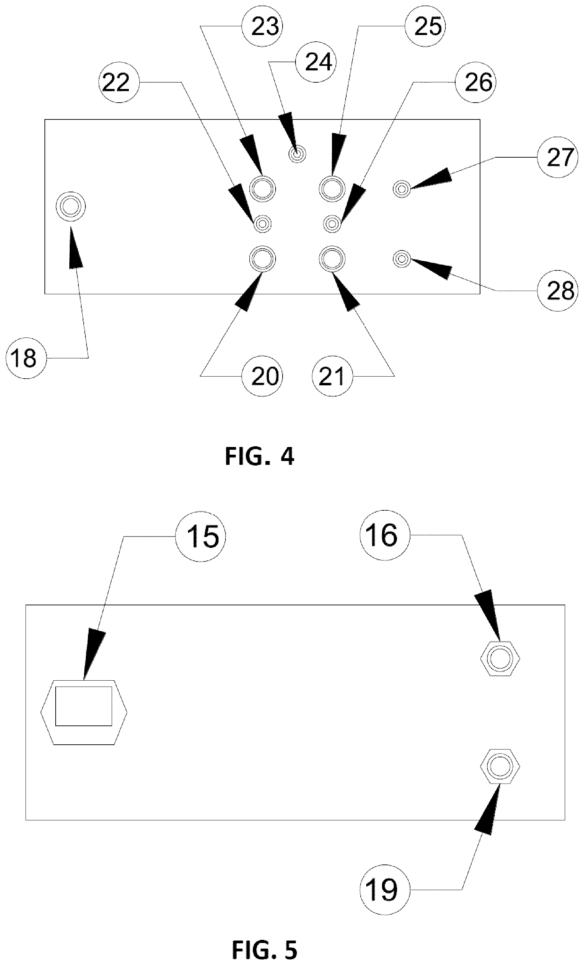

Figures 3 and 4 show the control panel 10 of the respirator according to the present invention and its indicators, while figure 5 shows the sockets of said control panel 10.

This control panel 10 includes a solenoid valve 11, which regulates the incoming air pressure, for example, to a maximum of 2 bars.

This solenoid valve 11 is controlled by a PLC (programmable logic controller) 12, which allows to regulate the passage, the time and, consequently, the volume of air supplied to the sealed pressure chamber 1.

The control panel 10 also comprises a one-way fine adjustment regulator 29 and two quick exhaust valves 7, which, when the solenoid valve 11 is switched via the PLC 12, depressurizes the quick exhaust valves 7, which open and automatically release the air from the pressure chamber 1 to the atmosphere, allowing the membrane 2 to return to its original shape, ready for the next cycle.

The elements that make up the control panel 10, together with the rest of the elements of the respirator, provide versatility for multiple combinations of number of cycles, duration of these cycles, intervals between cycles, etc., to be programmed according to requirements.

In the electrovalve 11 there is a pressure sensor 13 that checks at all times that we have pressure when we inject air into the membrane 2, that it will be compressed correctly, that it is not broken, and in general that we have the right pressure to start the cycle maneuvers.

The operation of the respirator according to the present invention is as follows.

First, an air inlet 19, e.g., 2 bars, is connected, which in the embodiment shown is located at the lower left rear of the control panel 10.

An air outlet 16, which in the illustrated embodiment is located at the top left rear of the control panel 10, and a power cable 15, located according to the illustrated embodiment at the center right rear of the control panel 10, are also connected to a power socket.

To start the respirator, a main switch 18 and a start button 23 must be actuated, and a light 24, e.g., green, will illuminate to inform the user.

The respirator will be started by cyclically compressing and decompressing the tubular membrane 2 through the fluid outlets and/or through the protrusions 9.

The respirator continues to operate until a stop button 25 is actuated, or a fault is detected. In the event that a fault is detected, an audible alarm 17 would sound and an alarm light 28 would illuminate.

If there is a need for more or less cycles per minute, there are the possibilities of, for example, 12 cycles per minute and 18 cycles per minute. To change the number of cycles per minute, you can increase or decrease the number of cycles per minute by pressing the appropriate switch without having to stop.

Thus, to select 12 cycles per minute, a switch 20 is pressed and a light 22 lights up, and to select 18 cycles per minute, a switch 21 is pressed and a light 26 lights up.

When in operation, a light 24, e.g., blue, is illuminated on the control panel 10 for each cycle performed by the respirator.

It should be noted that both options cannot be selected, as the respirator would stop and the audible alarm 17 would sound and the alarm light 28 would come on.

The alarms are expected to be for the following reasons:
- Lack of medical air supply;
- Membrane rupture 2;
- Double cycle selection.

After an alarm, to reset and restart, press stop button 25, then press run button 23. If no anomalies are detected, the respirator will start air cycling again.

By way of example only, the control panel 10 comprises the following elements on its front side:
18: Main power switch;
23: Push button, e.g., black;
25: Stop button, e.g., red;
24: Running lamp, e.g., green;
27: Respirator cycle indicator light, e.g., blue;
20: 12-cycle request switch to the respirator, e.g., yellow;
22: 12-cycle request indicator light, e.g., white;
21: 18-cycle request switch to the respirator, e.g., yellow;
26: 18-cycle request indicator light, e.g., white;
28: Breathing apparatus malfunction alarm lamp, e.g., orange.

By way of example only, the control panel 10 comprises the following elements on its rear side:
15: Fuse-protected socket outlet, e.g., 2 A, PC type;
19: Medical air inlet, maximum pressure, e.g., 2 bars;
16: Medical air outlet, for the respirator.

The following elements are arranged inside the respirator:
11: Solenoid valve;
12: Programmable PLC;
29: One-way controller;
13: Pressure sensor;
17: Audible alarm, triggered by an anomaly in the respirator;
14: PC socket outlet with built-in fuse, mains power cut-off switch.

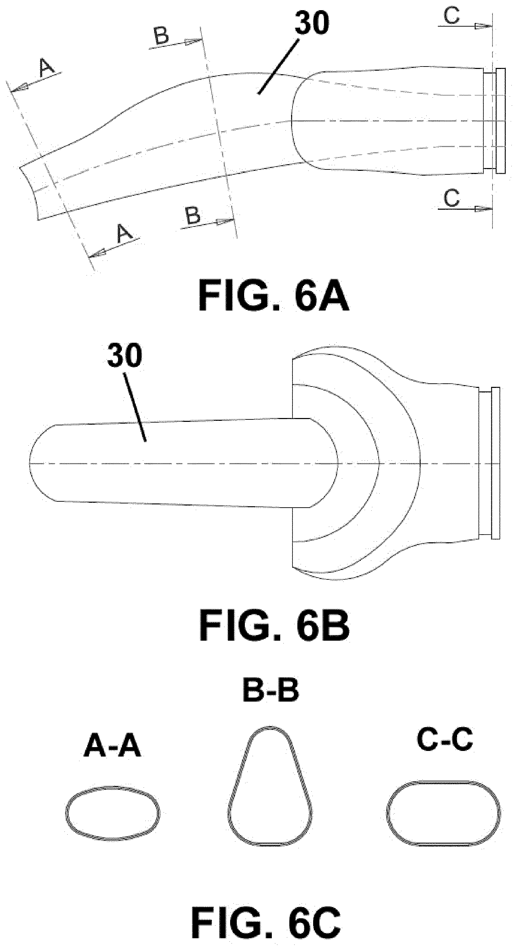

It should be noted that the respirator according to the present invention can be used with a cannula 30, an embodiment of which is shown in Figures 6A and 6B, with Figure 6C showing sections A-A, B-B and C-C of the cannula 30 indicated in Figure 6.

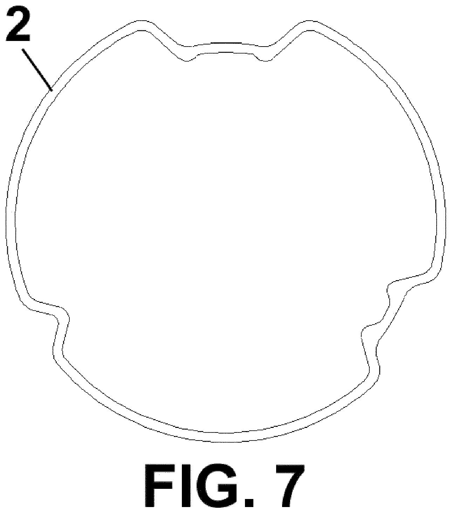

In addition, figure 7 shows a detail of the internal protrusions of the sealed chamber 1 that physically press by dimensional interference the membrane 2 of the AMBU so that the tricuspid collapse is orderly and sequential, not random.

Although reference has been made to a specific embodiment of the invention, it is obvious to a person skilled in the art that the respirator described is susceptible to numerous variations and modifications, and that all the details mentioned can be replaced by technically equivalent ones, without departing from the scope of protection defined by the appended claims.

## Claims

1. A respirator, comprising an air bag that is pressurized to provide air to a user, **characterized in that** the air bag is a tubular membrane (2) housed within a pressure chamber (1).

2. Respirator according to claim 1, wherein the pressure chamber (1) comprises fluid inlets and outlets, which fluid presses against the tubular membrane (2) to provide air to a user.

3. Respirator according to claim 1, wherein the pressure chamber (1) comprises protrusions (9) that press against the tubular membrane (2).

4. Respirator according to claims 2 and 3, wherein the fluid inlets and outlets are arranged in the protrusions (9) located inside the pressure chamber (1).

5. Respirator according to any one of the preceding claims, wherein the pressure chamber (1) comprises a flat membrane (5) as a seal and diaphragm.

6. Respirator according to claim 1, wherein the tubular membrane (2) comprises an upstream non-return valve (4) and a downstream non-return valve (4).

7. Respirator according to claim 1 or 5, wherein the pressure chamber (1) comprises one or more exhaust valves (7) for the intake of air into the pressure chamber (1) or for the exhaust of air from the pressure chamber (1).

8. Respirator according to any one of claims 1, 5 or 7, wherein the pressure chamber (1) comprises a support flange (6).

9. Respirator according to claim 1, wherein the pressure chamber (1) comprises flanges (3, 3.1) arranged to secure the tubular membrane (2) to the pressure chamber (1).

10. Respirator according to any one of the preceding claims, also comprising a control panel (10).

11. Respirator according to claim 10, wherein the control panel (10) comprises a solenoid valve (11) that regulates the air intake into the space between the tubular membrane (2) and the pressure chamber (1).

12. Respirator according to claim 11, wherein the solenoid valve (11) is connected to a programmable logic controller (12).

13. Respirator according to claim 10, wherein the control panel (10) comprises a pressure sensor (13).

14. Respirator according to any one of the preceding claims, comprising a portable power supply.

15. Respirator according to claim 14, wherein the portable power source is a compressed gas, medical air, or oxygen cylinder.

16. Respirator according to claim 3, wherein the protrusions (3) of the pressure chamber (1) physically press by dimensional interference against the tubular membrane (2), collapsing it into tricuspid in an orderly and sequential manner.
